# EUROPÄISCHE PATENTSCHRIFT

(11) **EP 0 316 668 B1**
(45) Veröffentlichungstag und Bekanntmachung des Hinweises auf die Patenterteilung: **19.05.1993**
(21) Anmeldenummer: 88118320.6
(22) Anmeldetag: 03.11.1988
(51) Int. Cl.: A61K 31/505

(54) **Verwendung eines Tetrahydro-pyrido(2,3-d)pyrimidins zur Herstellung eines Analgetikums**
Use of a tetrahydro-pyrido(2,3-d)pyrimidine for obtaining an analgetic agent
Utilisation d'une tetrahydro-pyrido(2,3-d)pyrimidine pour la préparation d'un agent analgèsique

(30) Priorität: 16.11.1987 DE 3738844
(43) Veröffentlichungstag der Anmeldung: 24.05.1989
(73) Patentinhaber: MERCK PATENT GmbH, D-64271 Darmstadt (DE)
(72) Erfinder: Raddatz, Peter, Dr., D-6100 Darmstadt (DE); Weber, Wolf-Dietrich, Dr., D-6107 Reinheim (DE); Barber, Andrew, Dr., D-6100 Darmstadt (DE); Wolf, Hans-Peter, Prof. Dr., D-6146 Alsbach-Hähnlein (DE); Seyfried, Christoph, Dr., D-6104 Jugenheim (DE)

(56) Entgegenhaltungen:
- EP-A- 0 013 612
- DE-A- 2 143 117
- DE-A- 3 601 731
- FR-A- 2 453 648
- GB-A- 843 073
- GB-A- 931 019
- US-A- 3 919 425
- EUROPEAN JOURNAL OF PHARMACOLOGY, Band 130, 1986, Seiten 311-314, Elsevier Science Publishers B.V.; G. SANDRINI et al.: "Evidence for serotonin-S2 receptor involvement in analgesia in humans"

## Beschreibung

Die Erfindung betrifft ein neues Analgetikum, enthaltend 3-[2-(4-p-Fluorbenzoylpiperidino)-ethyl]-2,4-dioxo-1,2,3,4-tetrahydro-pyrido[2,3-b]pyrimidin (I) oder eines seiner physiologisch unbedenklichen Salze.

Die Verbindung I und ihre Salze sind in der DE-A-36 01 731 beschrieben; jedoch finden sich dort keine Angaben über eine analgetische Wirkung.

Eine der Verbindung I konstitutionell ähnliche, jedoch schwächer analgetisch wirksame Verbindung ist das ketanserin (3-[2-(4-p-Fluorbenzoylpiperidino)-ethyl]-1,2,3,4-tetrahydrochinazolin-2,4-dion; vgl. EP-A-13612). Weitere, konstitutionell jedoch weniger ähnliche Substanzen sind beschrieben in DE-A-2 143 117, in Eur. J. Pharmacol., 1986, 130, 311-314, und in FR-A-2 453 648.

Der Erfindung lag die Aufgabe zugrunde, neue Analgetika mit hoher Wirksamkeit aufzufinden, insbesondere solche, die die unerwünschten Nebenwirkungen der bekannten Analgetika nicht oder nur in geringem Ausmaß zeigen.

Es wurde gefunden, daß die Verbindung I und ihre physiologisch unbedenklichen Salze analgetische Eigenschaften besitzen; so wirken die Verbindungen besonders stark im "Writhing-Tst" an Mäusen oder Ratten (Methode vgl. Siegmund, Cadmus und Golu, Proc. Soc. Exp. Biol. 95, (1957), 729-731). Die analgetische Wirkung läßt sich ferner im "Tail-flick-Test" an Mäusen oder Ratten nachweisen (Methodik vgl. d'Amour und Smith, J. Pharmacol, Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur).

Die Verbindungen können daher als Analgetika in der Human- und Veterinärmedizin verwendet werden.

Gegenstand der Erfindung ist die Verwendung der Verbindung I oder ihrer physiologisch unbedenklichen Säureadditionssalze zur Herstellung eines Analgetikums.

Einzelheiten der Herstellung der Verbindung I sind in der DE-A-36 01 731 beschrieben.

Als physiologisch unbedenkliche Säureadditionssalze der Base I eignen sich. Salze mit anorganischen Säuren, Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner mit organischen Säuren, insbesondere aliphatischen, alicyclischen, araliphatischen, aromatischen oder heterocyclischen ein- oder mehrbasigen Carbon-, Sulfon- oder Schwefelsäuren, z.B Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure.

Bei der Herstellung der erfindungsgemaßen Analgetika können die Verbindung I oder ihre physiologisch unbedenklichen Salze zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Die erfindungsgemäßen Analgetika können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindung I oder ihre physiologisch unbedenklichen Salze können bei der Bekämpfung von Schmerzzuständen verwendet werden.

Dabei werden diese Substanzen in der Regel in Analogie zu bekannten analgetisch wirksamen Stoffen wie Ketanserin, Ritanserin oder Guanethidin verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben.

### Herstellungsbeispiel

Ein Gemisch von 2,06 g 2-Ethoxycarbonylamino-nicotinsäure-ethylester (F. 60°; erhältlich durch 5std. Kochen von 2-Aminonicotinsäureethylester mit Chlorameisensäureethylester in Gegenwart von N,N-Diisopropylethylamin in Toluol) und 2,54 g 1-(2-Aminoethyl)-4-p-fluorbenzoyl-piperidin (Rf 0,43 an Kieselgel mit Butanol/Ethanol/Wasser/Ethylacetat/Essigsäure 50 : 50 : 50 : 25 : 25) wird 1 Std. auf 190° erhitzt. Man kühlt ab, nimmt in Methanol auf und erhält beim Abkühlen 3-[2-(4-p-Fluorbenzoylpiperidino)-ethyl]-2,4-dioxo-1,2,3,4-tetrahydro-pyrido[2,3-d]pyrimidin (I), F. 237°. Hydrochlorid, F. 297-298°. Fumarat, F. 253-255°.

### Beispiel: Kapseln

2 kg 3-[2-(4-p-Fluorbenzoylpiperidino)-ethyl]-2,4-dioxo-1,2,3,4,-tetrahydropyrimido[4,5-d]-pyrimidinhydrochlorid werden in üblicher Weise in Hartgelatinekapseln gefüllt, so daß jede Kapsel 20 mg des Wirkstoffs enthält.

## Patentansprüche

1. Verwendung von 3-[2-(4-p-Fluorbenzoylpiperidino)-ethyl]-2,4-dioxo-1,2,3,4-tetrahydro-pyrido[2,3-d]pyrimidin
oder von dessen physiologisch unbedenklichen Säureadditionssalzen zur Herstellung eines Analgetikums.

## Claims

1. Use of 3-[2-(4-p-fluorobenzoylpiperidino)-ethyl]-2,4-dioxol-1,2,3,4-tetrahydro-pyrido[2,3-d]pyrimidine or of its physiologically acceptable acid addition salts for the preparation of an analgesic.

## Revendications

1. Utilisation de la 3-[2-(4-p-fluorobenzoylpipéridino)-éthyl]-2,4-dioxo-1,2,3,4-tétrahydro-pyrido[2,3-d]pyrimidine ou de ses sels d'addition d'acides physiologiquement acceptables pour la préparation d'un analgésique.
